# EUROPEAN PATENT APPLICATION

(11) **EP 0 836 856 A2**
(43) Date of publication of application: **22.04.1998**
(21) Application number: 97102990.5
(22) Date of filing: 24.02.1997
(51) Int. Cl.: A61L 9/03

(54) **Fragrance burner body and fragrance burner**

(30) Priority: 17.10.1996 JP 10457/96
(71) Applicant: Nikko Industries Co., Ltd., Osaka City, Osaka Prefecture (JP)
(72) Inventor: Mitsuharu, Hashimoto, Kita-ku, Osaka City, Osaka Prefecture (JP); Hidehiko, Hashiba, Kita-ku, Osaka City, Osaka Prefecture (JP); Minoru, Onishi, Kita-ku, Osaka City, Osaka Prefecture (JP)
(74) Representative: Müller-Boré & Partner Patentanwälte

(57) **Abstract**

The fragrance burner of this invention includes: a fragrance burner body including a bowl for receiving an aromatic material and a ceramic semiconductor heater disposed on an outer bottom surface of the bowl for heating the bowl; and a support disposed removably for supporting the bowl of the fragrance burner body.

## Description

### BACKGROUND OF THE INVENTION

### 1. FIELD OF THE INVENTION:

The present invention relates to a fragrance burner body for evaporating an aromatic oil to fill the indoor atmosphere with incense or to be used for aroma therapy, and a fragrance burner including such a fragrance burner body.

### 2. DESCRIPTION OF THE RELATED ART:

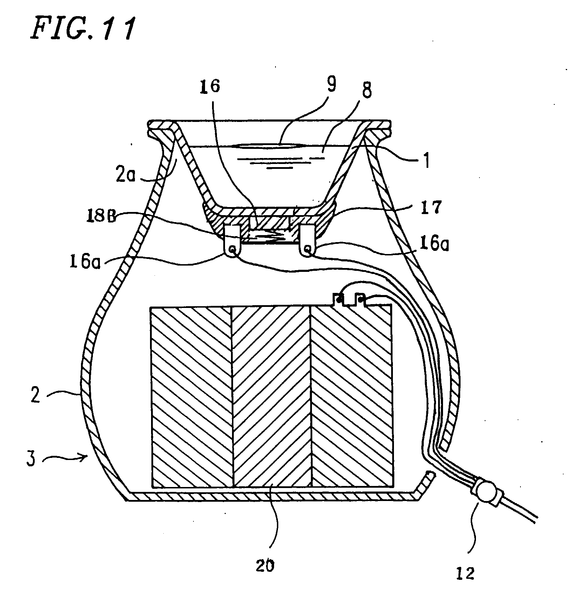

Figure **11** is a sectional view of a conventional fragrance burner. This fragrance burner includes a bowl **21** and a support **22** for supporting the bowl **21**. The support **22** is hollow inside, and a candle **23** is put inside the support **22** through an opening formed at a portion of the support **22**. An aromatic oil **25** such as an essential oil and a fragrant oil is put in the bowl **21** together with water **24** or hot water. The aromatic oil **25** evaporates by being heated with the flame of the candle **23** in the support **22**, emitting incense.

Such a conventional fragrance burner which uses a candle as the heating source is disadvantageous in the following points.

Since the size of the candle to be used is restricted depending on the size of the support of the fragrance burner body, the candle burns for only several hours at longest. Also, as the candle burns, the height of the position of the wick of the candle, as well as the size of the flame, change. This makes it difficult to control the burning of the candle. The environment where the fragrance burner is placed is also restricted since the flame of the candle may burn differently or even go out if the air stream abruptly changes. Moreover, the candle needs inconvenient manual operation whenever it is lit or put out.

### SUMMARY OF THE INVENTION

The fragrance burner body of this invention includes: a bowl for receiving an aromatic material; and a ceramic semiconductor heater disposed on an outer bottom surface of the bowl for heating the bowl.

In one embodiment of the invention, the ceramic semiconductor heater is provided with a radiation plate.

Alternatively, the fragrance burner body of this invention includes: a metal bowl for receiving an aromatic material; and a ceramic semiconductor heater disposed on an outer bottom surface of the bowl for heating the bowl, wherein the metal bowl serves as a radiation plate.

In one embodiment of the invention, the ceramic semiconductor heater is covered with a heat resistant material.

According to another aspect of the invention, a fragrance burner is provided. The fragrance burner includes: a fragrance burner body including a bowl for receiving an aromatic material and a ceramic semiconductor heater disposed on an outer bottom surface of the bowl for heating the bowl; and a support disposed removably for supporting the bowl of the fragrance burner body.

Alternatively, the fragrance burner of this invention includes: a fragrance burner body including a metal bowl for receiving an aromatic material and a ceramic semiconductor heater disposed on an outer bottom surface of the bowl for heating the bowl, the metal bowl serving as a radiation plate; and a support disposed removably for supporting the bowl of the fragrance burner body.

Alternatively, the fragrance burner of this invention includes: a bowl for receiving an aromatic material; a support disposed integrally with the bowl for supporting a periphery of the bowl; and a ceramic semiconductor heater disposed on an outer bottom surface of the bowl for heating the bowl.

Alternatively, the fragrance burner of this invention includes: a bowl for receiving an aromatic material; a support on which the bowl is placed; and a ceramic semiconductor heater disposed on a top surface of the support on which the bowl is placed, for heating the bowl.

In one embodiment of the invention, the ceramic semiconductor heater is provided with a radiation plate.

In another embodiment of the invention, the ceramic semiconductor heater is covered with a heat resistant material.

In still another embodiment of the invention, an indicator lamp indicating whether or not a voltage is being applied to the ceramic semiconductor heater is disposed at a position visible externally.

The fragrance burner of this invention includes: a bowl for receiving an aromatic material; a ceramic semiconductor heater disposed on an outer bottom surface of the bowl for heating; and a battery for supplying electric power to said heater.

Thus, the invention described herein makes possible the advantages of (1) providing a fragrance burner body capable of being placed in an environment which is less restrictive, being lit or put out simply and easily, and being used without time limit, and (2) providing a fragrance burner including such a fragrance burner body.

These and other advantages of the present invention will become apparent to those skilled in the art upon reading and understanding the following detailed description with reference to the accompanying figures.

### BRIEF DESCRIPTION OF THE DRAWINGS

Figure **1** is a sectional view of a fragrance burner of Example 1 according to the present invention.

Figures **2A** and **2B** are perspective views illustrating the steps of attaching a PTC heater to a bowl in Example 1.

Figures **3A** to **3D** show the PTC heater and a radiation plate in Example 1.

Figure **4** is a graph showing the relationships between the activating time and the temperature and between the activating time and the current for the fragrance burner of Example 1.

Figure **5A** is a sectional view of a fragrance burner body of Example 2 according to the present invention. Figures **5B** and **5C** are perspective views illustrating the steps of attaching a PTC heater to a bowl in Example 2.

Figure **6** is an exploded perspective view of a fragrance burner of Example 3 according to the present invention.

Figure **7** is a perspective view showing the appearance of the entire fragrance burner of Example 3.

Figures **8A** to **8C** show a fragrance burner of Example 4 according to the present invention, where Figure **8A** is an exploded perspective view showing the attachment of a PTC heater covered with a heat insulating sheath to a bowl, Figure **8B** is sectional view of the PTC heater covered with the heat insulating sheath, and Figure **8C** is an exploded perspective view showing the attachment of the bowl with the PTC heater attached thereto to a support.

Figure **9A** is an exploded perspective view of a fragrance burner of Example 5 according to the present invention. Figure **9B** is a sectional view of a support of the fragrance burner of Figure **9A**.

Figure **10** is a sectional view of a fragrance burner body of Example 6 according to the present invention.

Figure **11** is a sectional view of a fragrance burner of Example 7 according to the present invention.

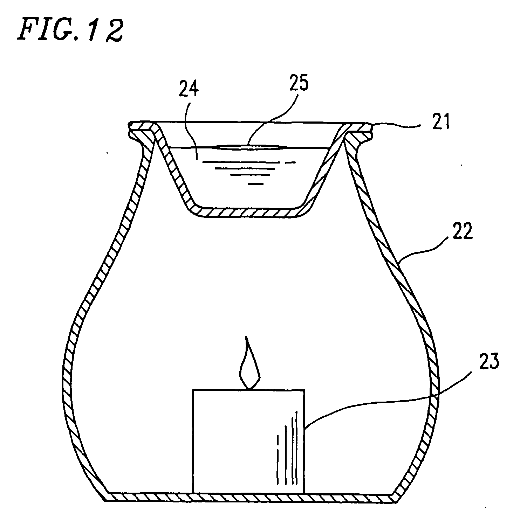

Figure **12** is a sectional view of a conventional fragrance burner.

### DESCRIPTION OF THE PREFERRED EMBODIMENTS

The fragrance burner of the present invention, which includes a censer, an incenser burner, and the like, uses a heater made of a ceramic semiconductor of a PTC (positive temperature coefficient) thermistor element as the heat energy supply source (hereinafter, such a heater is referred to as a PTC heater). Using the PTC heater, optimal heating conditions for evaporating an aromatic material in a bowl of the fragrance burner can be set by selecting the Curie point of the ceramic semiconductor. With this feature of the PTC heater, the temperature of the bowl can be kept constant at an optimal temperature, allowing the aromatic material in the bowl to be evaporated stably. Even after all the aromatic material has been evaporated, the temperature of the bowl can be kept constant. Therefore, the fragrance burner using the PTC heater is safe.

Besides the candle described above in the related art, a means of burning liquid fuel by use of a wick such as an alcohol lamp, solid fuel such as charcoal, a means of converting electricity into heat energy such as a Nichrome wire, and the like are also conventionally used as the heat energy supply source required for evaporating the aromatic material in the bowl. By any of these conventional means, however, it is difficult to continue to stably supply the small amount of heat energy required for evaporation by using a simple type of control. The cost to realize a stable supply will be high.

The PTC heater may be provided with a radiation plate which is disposed to be in contact with the bowl. With this arrangement, the heat from the PTC heater can be conducted uniformly in a wider area.

The PCT heater is preferably covered with a heat resistant resin, a heat resistant case, or the like made of a heat resistant material so that the heat of the PCT heater can be effectively conducted to the bowl.

The PCT heater provided with the radiation plate may be disposed in a support so that the radiation plate is exposed on the top surface of the support. The aromatic material in the bowl can be evaporated by placing the bowl on the radiation plate.

The radiation plate may be formed as the bowl, and the PTC heater is attached to the bottom surface of the bowl. This not only improves the heating efficiency but also reduces the number of components.

An indicator lamp showing whether or not a voltage is being applied to the terminals of the ceramic semiconductor heater is provided, so that whether or not the fragrance burner is under use can be confirmed by observing a light emitted from the indicator lamp. The user can therefore switch off the application of a voltage to the ceramic semiconductor heater if the user recognizes that the indicator lamp is lit when the user does not intend to use the fragrance burner. This saves wasteful power consumption.

The fragrance burner which includes a bowl for receiving an aromatic material, a ceramic semiconductor heater disposed on an out er bottom surface of the bowl for heating and a battery for supplying electric power to the heater, is also contained in this invention.

A primary battery for example, manganese battery, alkali-manganese battery and other kinds of dry-battery are available for the above-mentioned fragrance burner. Furthermore, a secondary battery for example, lead-sulfate battery, nickel-cadmium battery, nickel-hydrogen battery, lithium-ion battery and other kinds of re-chargeable type battery are available for the fragrance burner. Among them, a nickel-hydrogen battery and lithium-ion battery are more preferable because their efficiency of power out put per volume are higher than other type of secondary batteries.

In this type of the fragrance burner, the semiconductor heater is heated by an electric power of thw battery, however, it is possible to heat the heater directly only by ordinary electric souce of an alternating current, without that of battery.

The bowl and the support of the fragrance burner may be inseparably integrated or separable. These types of fragrance burners are manufactured in different ways but are not especially different from each other when used.

Hereinbelow, the present invention will be described by way of examples with reference to the accompanying drawings.

### (Example 1)

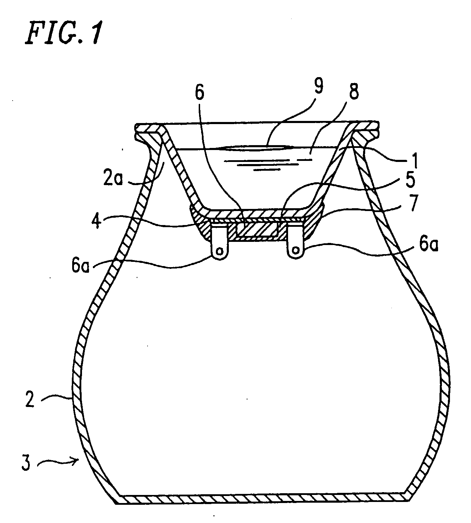

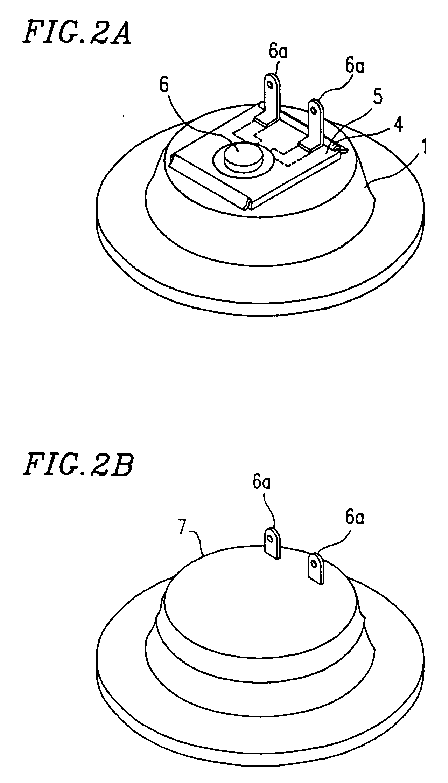

Figure **1** is a sectional view of a fragrance burner of Example 1 according to the present invention. Figures **2A** and **2B** are perspective views illustrating the steps of attaching a PTC heater to a bowl in this example.

A fragrance burner **3** of this example includes a bowl **1** and a support **2** for supporting the periphery of the bowl **1**. An aromatic material containing water **8** or hot water and an aromatic oil **9** such as an essential oil and a fragrant oil is placed in the bowl **1**. The support **2** is hollow inside, and has a plurality of holes (not shown) formed through the wall thereof to ventilate the inside of the support **2**. An opening **2a** is formed at the top of the support **2** to receive the bottom portion of the bowl **1**. The periphery of the opening **2a** supports the periphery of the bowl **1**.

As shown in Figures **1** and **2A**, a PTC heater **6** provided with a radiation plate **5** is bonded to the bottom surface of the bowl **1** constituting the fragrance burner **3** with a silicone adhesive **4** so that the radiation plate **5** is in contact with the bottom surface of the bowl **1**. More specifically, the radiation plate **5** is thinly coated with a material with good heat conduction such as silicone grease, and closely bonded to the bottom surface of the bowl **1** so that no air layer is interposed therebetween. Then, the silicone adhesive **4** is applied to the peripheral portions of the radiation plate **5** on the bottom surface of the bowl **1**, and cured to fix the radiation plate **5** to the bowl **1**, forming ridge-shaped adhesive portions at the peripheries of the radiation plate **5** on the bottom surface of the bowl **1**. As the adhesive **4**, inorganic adhesives and any other adhesives which have heat resistance and an bond strength large enough to endure actual use can be used. Alternatively, the PTC heater **6** may be mechanically attached to the bowl **1** by screwing the radiation plate **5** on the bowl **1**.

The bottom surface of the bowl **1** should have a high flatness by polishing and the like to enhance the bonding between the bowl **1** and the radiation plate **5**. The radiation plate **5** may be made of metal such as aluminum and copper or ceramic having high heat conduction such as alumina and beryllia. The radiation plate **5** is disposed to efficiently conduct heat generated by the PTC heater **6** to the bowl **1**. The radiation plate **5** may be omitted. In such a case, a material with high heat conduction such as silicone grease as described above is preferably applied between the PTC heater **6** and the bowl **1**.

After the attachment of the PTC heater **6** to the bowl **1**, the entire PTC heater **6** including the radiation plate **5** is covered with a heat resistant material **7** as shown in Figure **2b**. As the heat resistant material **7**, silicone RTV rubber, resin, varnish, epoxy resin, inorganic materials such as alumina cement, and the like may be used.

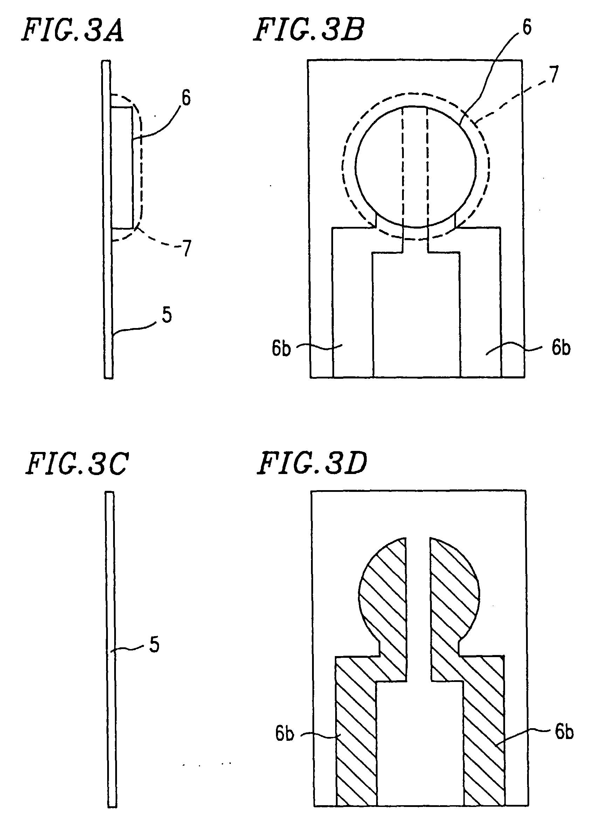

Two terminals **6a** of the PTC heater **6** extend from the heat resistant material **7** and are connected to an end of a cord with a plug disposed at the other end. The two terminals **6a** are disposed for two electrodes **6b** formed on the surface of the radiation plate **5** in consideration of the position of the PTC heater **6**, as shown in Figures **3B** and **3D**. A remote switch may be provided somewhere on the cord to facilitate the turning on/off of the PTC heater **6**. Figure **3A** is a side view showing the radiation plate **5** and the PTC heater **6**, Figure **3B** is a front view of Figure **3A**, Figure **3C** is a side view showing only the radiation plate **5**, and Figure **3D** is a front view of Figure **3C**.

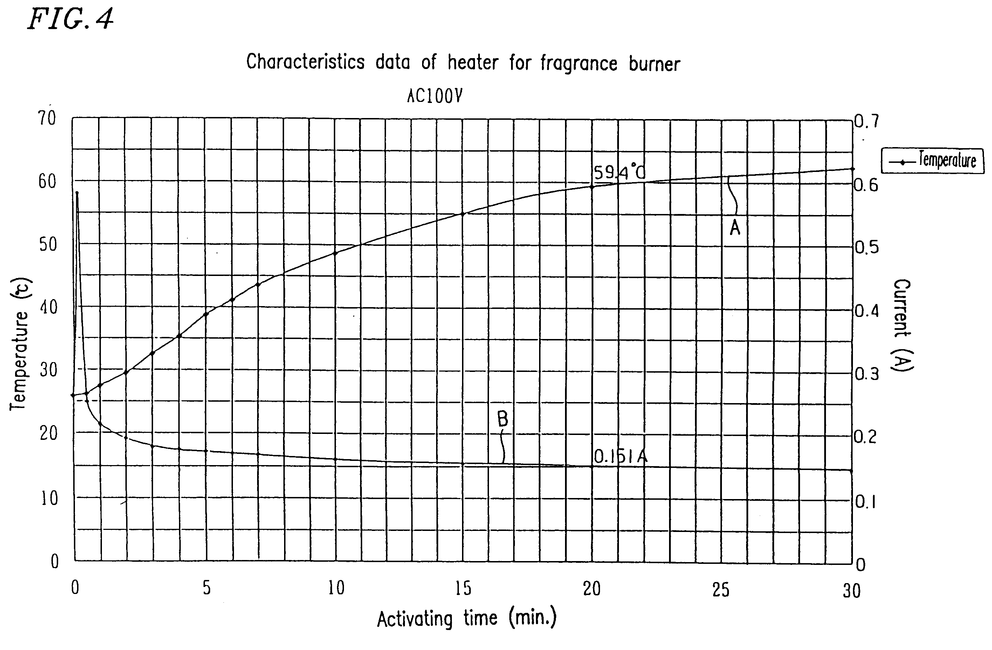

Figure **4** is a graph showing the relationships between the activating time (in minutes) and the temperature (°C) and between the activating time and the current for the fragrance burner of this example. Curve **A** represents the temperature while curve **B** represents the current. A PTC heater with a Curie point of 160°C was used in this measurement.

As is observed from Figure **4**, the temperature of the aromatic material in the bowl was kept constant in the range of 60 to 63°C after the heater was activated. The power consumption during this constant temperature was as small as about 15 W. This is economically advantageous.

A voltage of 200 V or 100 V may be applied to the fragrance burner of Example 1. An indicator lamp showing the status of the voltage applied to the ceramic semiconductor heater is preferably disposed on the support **2**. The bowl **2** is preferably removable from the support **2** so that it can be cleaned.

### (Example 2)

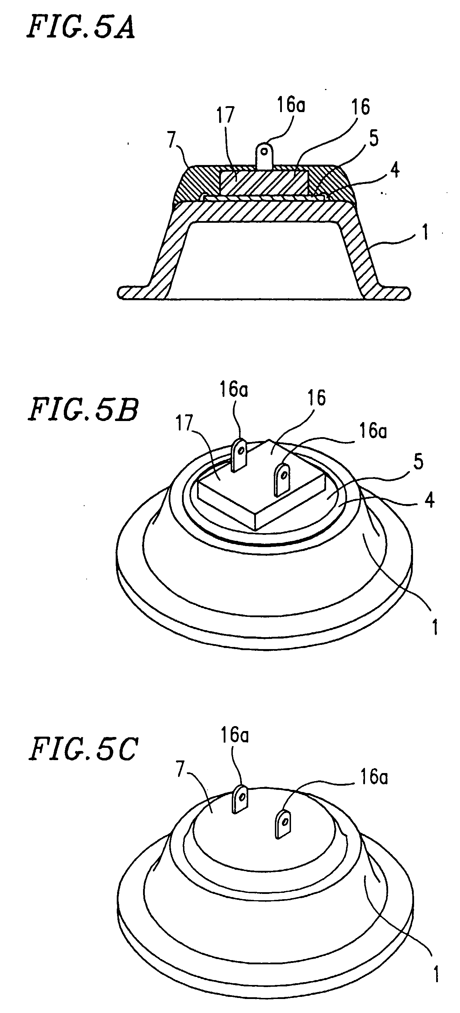

Figure **5A** is a sectional view of a fragrance burner body of Example 2 according to the present invention. Figures **5B** and **5C** are perspective views illustrating the steps of attaching a PTC heater to a bowl. The same components as those shown in Figure **1** are denoted by the same reference numerals.

In this example, a PTC heater **16** is covered with a heat insulating sheath **17**. Terminals **16a** extend from the sheath **17**. The terminals **16a** also extend from a heat resistant material **7** when the PTC heater **16** is further covered with the heat resistant material **7** as shown in Figure **5C**. The exposed terminals **16a** are connected to an end of a cord as in Example 1.

The fragrance burner body of this example with the above structure may be placed on a support which supports the periphery of the bowl **1**, to serve as a fragrance burner.

Such a support is preferably a pot-shaped vessel having a hollow inside configured to receive the bowl **1** except for the periphery thereof so that the heat insulating sheath **17** covering the PTC heater **16** is invisible from outside of the support.

Alternatively, the inner bottom of the bowl **1** may be raised, and the PTC heater **16** may be disposed in the space formed by the raised bottom so that the heat insulating sheath **17** covering the PTC heater **16** is invisible externally. With this structure, no support is necessary, or legs and the like may be attached to the bowl **1**.

A voltage of 200 V or 100 V may be applied to the fragrance burner body of Example 2. An indicator lamp showing the status of the voltage applied to the ceramic semiconductor heater is preferably disposed on the bowl.

### (Example 3)

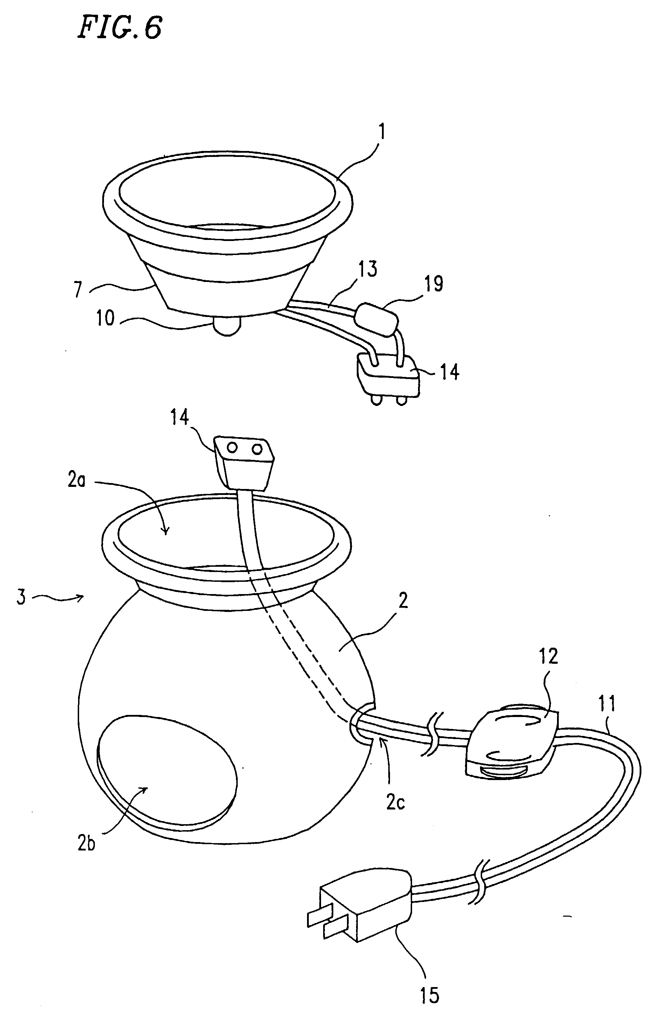

Figure **6** is an exploded perspective view of a fragrance burner of Example 3 according to the present invention, showing a bowl **1** and a support **2** separately.

In this example, a PTC heater covered with a heat insulating sheath as in Example 2 is attached to a radiation plate, and the radiation plate is bonded to the bottom surface of the bowl **1** as described in Example 1. A heat resistant material **7** covers the heat insulating sheath. In the fragrance burner of this example, an indicator lamp **10** for indicating that the PTC heater is in the state of being applied with a voltage (ON state) is disposed on the heat resistant material **7**. The indicator lamp **10** does not respond to the automatic on/off operation by the PTC heater itself serving as a thermistor, but lights up only when a remote switch **12** disposed somewhere on a cord **11** extending through a hole **2c** of a support **2** is turned on. More specifically, the indicator lamp **10** includes a tinted neon bulb, to which a voltage is applied via a wire extending from a terminal (not shown) of the PTC heater directly or from a portion adjacent to the terminal. A resistor may be disposed on the wire if necessary for the lighting of the neon bulb.

A pair of pin connectors **14** are disposed at one end of a cord **13** the other end of which is connected to the terminal (not shown) of the PTC heater and at one end of the cord **11** on the side of the PTC heater. The pair of pin connectors **14** are connected together, and a plug **15** disposed at the other end of the cord **11** is connected to a home outlet (e.g., 100V or 200 V) to allow a voltage to be applied to the PCT heater. The PTC heater can be turned on/off with the remote switch **12** on the cord **11** while the plug **15** is kept connected to the home outlet. A fuse **19** is disposed on the cord **13** to discontinue a current flow if an abnormality arises.

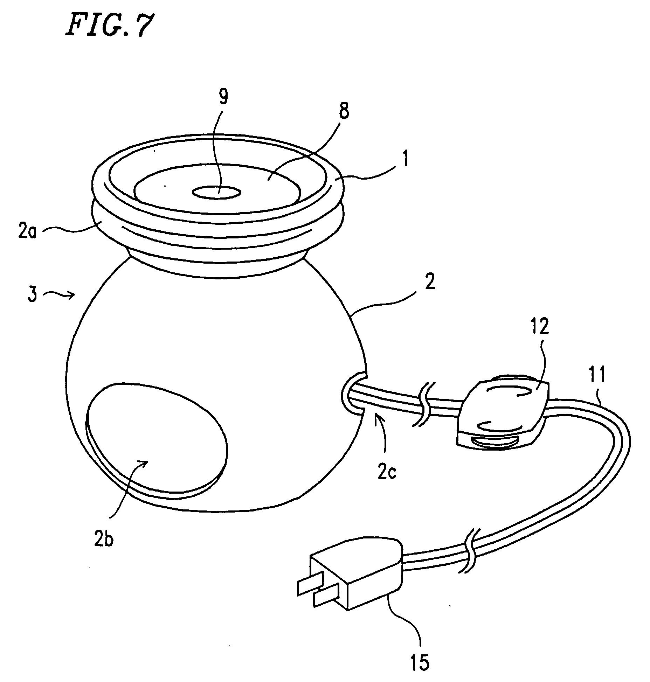

An opening **2b** is formed at an appropriate position of the support **2** to allow light emitted from the indicator lamp **8** to be visible externally when the bowl **1** is placed in the support **2** through an opening **2a** with the periphery of the bowl **1** being supported on the periphery of the support **2**. Thus, the user can confirm that the remote switch **12** is in the ON state by observing the lighting of the indicator lamp **10** via the opening **2b**. The ON state of the remote switch **12** also means that the fragrance burner, i.e., the heater, is under heating. Figure **7** is a perspective view of the entire fragrance burner of this example.

The surface of the fragrance burner **3** composed of the bowl **1** and the support **2** may be patterned with a plurality of colors to enhance the appearance. The bowl **1** is removably placed on the support **2**.

### (Example 4)

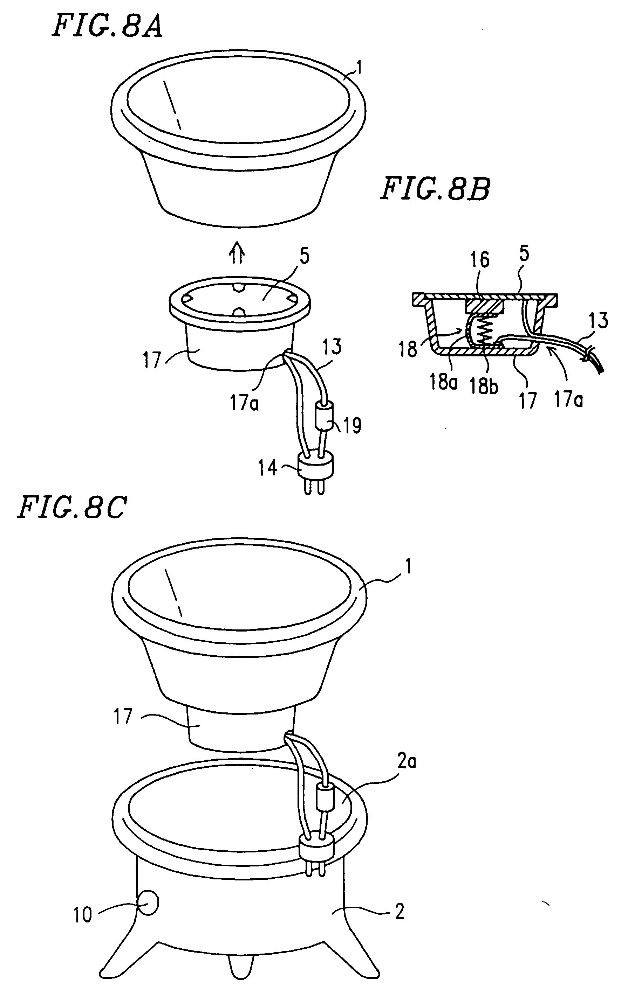

A fragrance burner of Example 4 will be described with reference to Figures **8A** to **8C**. Figure **8A** is an exploded perspective view illustrating the attachment of a PTC heater covered with a heat insulating sheath to a bowl. Figure **8B** is a sectional view of the PTC heater covered with the heat insulating sheath. Figure **8C** is an exploded perspective view illustrating the attachment of the bowl with the PTC heater attached thereto to a support.

As shown in Figures **8A** and **8B**, a PTC heater **16** is bonded to a disk-shaped radiation plate **5**. The bonding of the PTC heater **16** to the radiation plate **5** is further secured by a press member **18** disposed between the PTC heater **16** and a heat insulating sheath **17** made of heat resistant plastic and the like. The press member **18** includes a bent element **18a** formed by bending a comparatively flexible material into a C shape and a spring **18b** extending between the two ends of the bent element **18a**. The heat insulating sheath **17** has a hole **17a** for allowing a cord **13** to extend therethrough. A voltage is applied to the PTC heater **16** through the cord **13**. The voltage applied to the PTC heater **16** may be 200 V or 100 V.

A bowl **1** with the PTC heater **16** attached thereto is placed in a support **2** through an opening **2a** with the periphery of the bowl **1** being supported on the periphery of the support **2**, as shown in Figure **8C**. An indicator lamp **10** is disposed on the circumferential surface of the support **2** so that the ON state of the PTC heater **16** can be confirmed externally as in Example 3. In this example, the, indicator lamp **10** is connected to the PTC heater **16** via a cord with an appropriate length, and a voltage applied to the PTC heater **16** is applied to the indicator lamp **10**. The inner depth of the support **2** is comparatively small, and is substantially the total of the height of the heat insulating sheath **17** and the height of the bowl **1**, i.e., the distance between the bottom of the periphery and the bottom surface of the bowl **1**. Such a low support **2** is possible because the PTC heater **16** is used instead of a candle. Therefore, the height of the entire fragrance burner can be lowered to a level which has not been conventionally achieved.

### (Example 5)

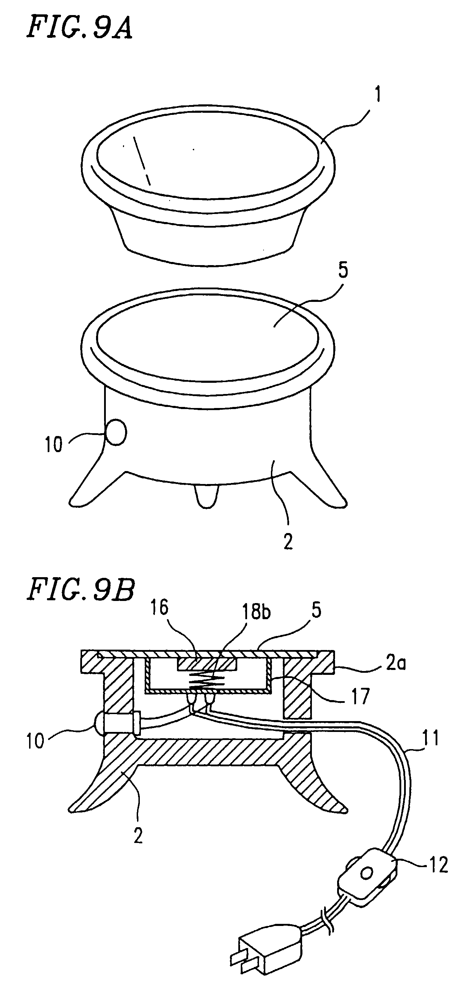

Figure **9A** is an exploded perspective view of a fragrance burner of Example 5, and Figure **9B** is a sectional view of a support of the fragrance burner of this example. In this example, a radiation plate **5** and a PTC heater **16** are disposed in a support **2**, and a bowl **1** is placed on the support **2**.

More specifically, the periphery of the radiation plate **5** is seated on the periphery of an opening **2a** of the support **2** to fix the radiation plate **5** on the support **2**. The PTC heater **16** is bonded to the radiation plate **5** fixed to the support **2**. A spring **18b** is disposed between the PTC heater **16** and a heat insulating sheath **17** fixed to the radiation heat **5** in a state of incomplete expansion. A cord **11** with a remote switch **12** disposed thereon is connected to the PTC heater **16**. The other configuration is the same as that of Example 4. An indicator lamp **10** is disposed on the support **2**. The indicator lamp **10** lights up when the remote switch **12** is in the ON state and goes out when it is in the OFF state.

Thus, according to the fragrance burner of this example, the bowl **1** can be heated by placing the bowl **1** on the top surface of the support **2** where the radiation plate **5** is exposed.

A voltage of 200 V or 100 V may be applied to the PTC heater **16**.

### (Example 6)

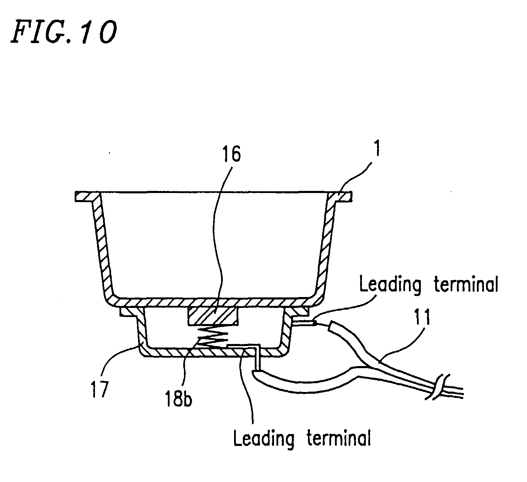

Figure **10** is a sectional view of a fragrance burner body of Example 6 according to the present invention.

The fragrance burner component of this example includes a bowl **1** made of metal which can serve as the radiation plate. A PTC heater **16** covered with a heat insulating sheath **17** is bonded to the bottom surface of the bowl **1**. A spring **18b** is disposed between the PTC heater **16** and the heat insulating sheath **17**. The heat insulating sheath **17** is fixed to the metal bowl **1** by screwing, to ensure the fixation of the PTC heater **16** on the bottom surface of the bowl **1**. A voltage of 200 V or 100 V may be applied to the PTC heater **16**.

The fragrance burner body of this example can be placed in an appropriate support through an opening, with the periphery of the fragrance burner body being supported on the periphery of the support, to complete a fragrance burner. The user can select a support with a desirable shape and appearance according to the preference of the user, to complete his or her own unique fragrance burner.

Using the fragrance burner body of this example, the heat of the PTC heater **16** can be directly conducted to the metal bowl where an aromatic material is placed. This provides a higher heating efficiency than those obtained in the above examples, reducing power consumption.

### (Example 7)

Figure **11** is a sectional view of a fragrance burner of Example 7 according to the present invention.

The fragrance burner component of this example includes a bowl 1 made of metal which can serve as the ra diation plate. A PTC heater 16 covered with a heat insulating shearth 17, is bonded to the bottom surface of the bowl 1. A spring 18B is disposed between the PTC heater 16 and the heat insulating shearth 17. The heat insulating shearth 17 is fixed to the metal bowl 1 by screwing, to ensure the fixation of the PTC heater 16 on the bottom surface of the bowl 1.

Sometimes, a thin mica plate is used in stead of a metal bowl and a tip of a fragrant wood is put directly on the mica plate.

A litium ion battery 20 is equipped inside the support or case of the bowl 1 and leading terminals of the battery are connected with these of the PTC heater 16 through switch and other additional devices. Which electric source of the battery or an ordinary alternating current is available depend on the case. the litium ion battery sometimes contains a voltage adjustment device in it.

Thus, according to the present invention, by using the PTC heater as the heating source, the temperature of the content in the bowl can be kept constant, and the optimal conditions for stably evaporating water containing an aromatic oil in the bowl can be maintained. Moreover, according to the fragrance burner of the present invention, the environment where the fragrance burner is placed is less restrictive. The fragrance burner can be simply and easily lit or put out, and can be used without time limit. Any temperature required for the evaporation of a content in the bowl can be set by selecting the Curie point of the PTC heater.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. A fragrance burner body comprising:
a bowl (1) for receiving an aromatic material (9); and
a ceramic semiconductor heater (6; 16) disposed on an outer bottom surface of the bowl (1) for heating the bowl (1).

2. A fragrance burner body according to claim 1, wherein the bowl is a metal bowl (1) and wherein the metal bowl (1) serves as a radiation plate.

3. A fragrance burner comprising:
a fragrance burner body including a bowl (1) for receiving an aromatic material (9) and a ceramic semiconductor heater (6; 16) disposed on an outer bottom surface of the bowl (1) for heating the bowl; and
a support (2) disposed removably for supporting the bowl (1) of the fragrance burner body.

4. A fragrance burner according to claim 3, wherein the bowl (1) is the metal bowl, the metal bowl serving as a radiation plate.

5. A fragrance burner comprising:
a bowl (1) for receiving an aromatic material (9);
a support (2) disposed integrally with the bowl (1) for supporting a periphery of the bowl (1); and
a ceramic semiconductor heater (6; 16) disposed on an outer bottom surface of the bowl (1) for heating the bowl (1).

6. A fragrance burner comprising:
a bowl (1) for receiving an aromatic material (9);
a support (2) on which the bowl (1) is placed; and
a ceramic semiconductor heater (6; 16) disposed on a top surface of the support (2) on which the bowl (1) is placed, for heating the bowl (1).

7. A fragrance burner according to any of the preceding claims, wherein the ceramic semiconductor heater (6; 16) is provided with a radiation plate (5).

8. A fragrance burner according to any of the preceding claims, wherein the ceramic semiconductor heater (6; 16) is covered with a heat resistant material (7).

9. A fragrance burner according to any of the preceding claims, wherein an indicator lamp (10) indicating whether or not a voltage is being applied to the ceramic semiconductor heater (6; 16) is disposed at a position visible externally.

10. A fragrance burner according to any of the preceding claims comprising a battery (20) for supplying electric power to said heater.
